# EUROPÄISCHE PATENTSCHRIFT

(11) **EP 3 436 832 B1**
(45) Veröffentlichungstag und Bekanntmachung des Hinweises auf die Patenterteilung: **03.08.2022**
(21) Anmeldenummer: 17714403.7
(22) Anmeldetag: 16.03.2017
(51) Int. Cl.: G01N 35/00, C12M 1/22, C12M 1/00

(54) **FÖRDERVORRICHTUNG**
CONVEYING DEVICE
DISPOSITIF DE TRANSPORT

(30) Priorität: 29.03.2016 DE 102016105683
(43) Veröffentlichungstag der Anmeldung: 06.02.2019
(73) Patentinhaber: Andreas Hettich GmbH & Co. KG, 78532 Tuttlingen (DE)
(72) Erfinder: EBERLE, Klaus-Günter, 78532 Tuttlingen (DE)
(74) Vertreter: Puschmann Borchert Kaiser Klettner Patentanwälte Partnerschaft mbB
(86) Internationale Anmeldenummer: PCT/EP2017/056305
(87) Internationale Veröffentlichungsnummer: WO 2017/167585

(56) Entgegenhaltungen:
- WO-A1-2008/083440
- DE-A1-102010 044 125
- JP-A- H06 225 753
- US-A- 4 572 067
- US-A1- 2011 243 814
- US-A1- 2014 030 802

## Beschreibung

Die Erfindung betrifft eine Fördervorrichtung zur Positionierung und Bereitstellung von Laborgefäßen für Proben, Mikroorganismen, Zellkulturen oder ähnlichem für die Analyse, Probenpräparation und/oder Probenmanipulation an einem zugeordneten Gerät gemäß der im Oberbegriff des Patentanspruches 1 angegebenen Art sowie ein Verfahren zur Positionierung und Bereitstellung von Laborgefäßen für Proben mit der Fördervorrichtung gemäß dem Patentanspruch 11.

Im Stand der Technik sind Fördereinheiten in Vorrichtungen für die Analyse- oder Verarbeitung von biologischem Material bekannt, bei denen Greifer eingesetzt werden, die Laborgefäße, meist in Stapeln angeordnete Petrischalen, in einem Eingangsbereich einzeln aufnehmen und in einen Analysebereich fördern. Während der Analyse hält der Greifer die Petrischale und fördert sie anschließend, abhängig vom Aufbau der Vorrichtung, entweder zurück in den Eingangsbereich oder weiter in einen eigens vorgesehenen Ausgangsbereich.

Dazu bewegen Aktuatoren einen einzelnen Greifer entlang mehrerer Achsen, sowohl geradlinig als auch rotatorisch. Dies ist konstruktiv leicht und mit vergleichsweise geringem finanziellem Aufwand lösbar.

Je größer die Anzahl der Achsen ist, entlang derer der Greifer bewegt wird, desto geringer wird jedoch der Grad der Präzision der Positionierung. Allerdings können bereits Abweichungen von wenigen Millimetern von der Position, in die eine Petrischale zur Analyse gebracht werden muss, zu ungenauen Ergebnissen führen.

Eine Fördervorrichtung ist aus der US 2016/0083686 A1 bekannt. Die Fördervorrichtung weist zumindest eine erste Fördereinheit zum Zu- und/oder Abfördern der Laborgefäße aus einem Ausgangsbereich zu einem Bereitstellungsbereich auf, wo das Laborgefäß für die Analyse oder Präparation gehalten wird. Dabei sind mehrere Fördereinheiten vorhanden. welche jeweils lediglich eine translatorische Bewegung des Laborgefäßes entlang einer Achse durchführen. Die erste Fördereinheit fördert dabei das Laborgefäß aus dem Ausgangsbereich zu einem vorbestimmten Höhenbereich und vice versa vertikal. Eine zweite Fördereinheit ist vorgesehen, welche das Laborgefäß aus dem Höhenbereich in den Bereitstellungsbereich und vice versa horizontal fördert.

Eine gattungsgemäße Fördervorrichtung ist aus der WO 2008/083440 A1 bekannt. Die Fördervorrichtung umfasst eine erste Fördereinheit zum Zu- und/oder Abfördern der Laborgefäße aus einem Ausgangsbereich zu einem Bereitstellungsbereich. Die erste Fördereinheit fördert das Laborgefäß vertikal aus dem Ausgangsbereich zu einem vorbestimmten Höhenbereich und vice versa. Eine zweite Fördereinheit ist vorgesehen, welche das Laborgefäß in den Bereitstellungsbereich und vice versa horizontal fördert, wobei die Fördereinheiten jeweils lediglich eine translatorische Bewegung des Laborgefäßes entlang einer Achse durchführen.

Nachteilig an den bekannten Fördervorrichtungen ist, dass die Ausrichtung zum Analysegerät nicht exakt genug ist. Hierdurch werden die Ergebnisse verfälscht oder es sind zeitraubende Korrekturbewegungen zum Ausrichten zum Analysegerät notwendig.

Aufgabe der Erfindung ist es, unter Vermeidung der genannten Nachteile eine Fördervorrichtung zur Positionierung und Bereitstellung von Laborgefäßen derart weiterzubilden, dass eine exakte Positionierung von Laborgefäßen in beliebig vielen Durchgängen gewährleistet ist.

Diese Aufgabe wird durch die kennzeichnenden Merkmale des Patentanspruches 1 in Verbindung mit seinen Oberbegriffsmerkmalen gelöst.

Die Unteransprüche bilden vorteilhafte Weiterbildungen der Erfindung.

Der Erfindung liegt die Erkenntnis zugrunde, mehrere seriell angeordnete Aktuatoren, welche jeweils eine einzelne Bewegung entlang einer Achse durchführen, statt eines mehrachsigen Aktuators, der die komplette Bewegung durchführt, zu verwenden. Dadurch kann das Laborgefäß exakter relativ zum Analysegerät angeordnet und gehalten werden. Zudem können zusätzliche Maßnahmen zur Überprüfung und Verbesserung der Ausrichtung und Anordnung zwischengeschaltet werden. Die Flexibilität und Präzision des Systems erhöht sich. Neben der präzisen Führung durch die Fördereinheiten wurde weiter erkannt, dass die Bewegungsabläufe dann mit hoher Präzison verlaufen und kaum eine Korrekturbewegung im Bereitstellungsbereich notwendig ist, wenn das Laborgefäß zu Beginn der Förderbewegung aus dem Ausgangsbereich heraus bereits präzise zur Achse der ersten Fördereinheit ausgerichtet ist.

Gemäß der erfindungsgemäßen Ausgestaltung fördert die zweite Fördereinheit aus dem Höhenbereich das Laborgefäß in den Bereitstellungsbereich und vice versa horizontal. Im Bereitstellungsbereich wird das Laborgefäß dann von der zweiten Fördereinheit für die Analyse oder Präparation gehalten.. Dadurch, dass die vertikale Förderung und die horizontale Förderung verschiedenen Aktuatoren zugeordnet sind, kann die Präzision der Positionierung des Laborgefäßes deutlich erhöht werden. So sind deutlich exaktere und zuverlässigere Ergebnisse bei der nachfolgenden Analyse oder Präparation zu erwarten, da das Laborgefäß exakt zum Gerät ausgerichtet ist.

Zudem ist eine Zentriervorrichtung vorgesehen, welche das Laborgefäß relativ zur Achse der ersten Fördereinheit mittig zentriert. So ist das Laborgefäß zum Zeitpunkt der Übergabe von der ersten Fördereinheit an die zweite Fördereinheit bereits vorzentriert, und durch die zweite Fördereinheit müssen gegebenenfalls nur noch kleine Korrekturen in der Positionierung des Laborgefäßes vorgenommen werden, damit das Laborgefäß im Bereitstellungsbereich exakt in der für die Analyse erforderlichen Position gehalten wird. Dadurch wird die Leistung der Fördervorrichtung nochmals verbessert.

Die erste Fördereinheit weist dabei ein Auflager für das Laborgefäß auf, das horizontal ausgerichtet ist, und die Achse der ersten Fördereinheit erstreckt sich hierzu vertikal. Beispielsweise kann von einer dritten Fördereinheit das Laborgefäß horizontal so weit bewegt werden, bis es im Ausgangsbereich auf dem Auflager der ersten Fördereinheit lagert. Durch eine Bewegung der ersten Fördereinheit gelangt das Laborgefäß auf dem Auflager dann in den vorbestimmten Höhenbereich, wo es von der zweiten Fördereinheit übernommen wird. Diese Lösung ist einfach und kostengünstig.

Des Weiteren umfasst die Zentriervorrichtung einen Rotationsantrieb für das Auflager und eine seitliche Ringführung für das Laborgefäß, welche sich konisch in eine Richtung verkleinert. Zur Zentrierung des Laborgefäßes verfährt das Auflager mit dem Laborgefäß in die Richtung, in der sich die konische Ringführung verkleinert. Dabei ist der Innendurchmesser der Ringführung so bemessen, dass er an der Stelle seiner maximalen Ausdehnung größer ist als der Durchmesser des größten zu verwendenden Laborgefäßes. An seiner geringsten Stelle ist der Innendurchmesser der Ringführung kleiner als der Durchmesser des kleinsten zu verwendenden Laborgefäßes, so dass das Laborgefäß zum Ende des Zentriervorgangs auf der Ringführung lagert. Die Rotation des Auflagers während der Bewegung in die Richtung, in der sich die konische Ringführung verkleinert, wirkt dabei einem Verkanten und insbesondere einer Schieflage des Laborgefäßes in der Ringführung entgegen. Dadurch wird auf einfache Art und Weise eine schnelle und sichere Zentrierung und somit exakte Ausrichtung des Laborgefäßes zu dem Gerät für die Analyse oder Präparation erreicht.

Bei einer vorteilhaften Weiterbildung der Erfindung ist eine dritte Fördereinheit vorgesehen, welche ein Laborgefäß aus einem Eingabebereich zu dem Ausgangsbereich horizontal fördert. Dies ermöglicht es, den Eingabebereich beispielsweise in einem größeren Abstand zum Ausgangsbereich anzuordnen, woraus sich eine größere Flexibilität bei der Eingabe der Laborgefäße ergibt.

Günstig ist es, wenn zudem eine vierte Fördereinheit vorgesehen ist, welche ein Laborgefäß aus dem Ausgangsbereich zu einem Ausgabebereich horizontal fördert. Durch die Bereitstellung eines vom Eingabebereich entfernten Ausgabebereichs werden höhere Ladekapazitäten von Laborgefäßen erreicht. Ferner ist prinzipiell eine kontinuierliche Förderung und Analyse möglich, da Eingabe von Laborgefäßen in die Fördervorrichtung und Ausgabe aus der Fördervorrichtung unabhängig voneinander sind. Die Leistung der Fördervorrichtung wird somit effizienter.

Vorzugsweise ist das Auflager nach unten und oben entlang der vertikal ausgerichteten Achse verfahrbar. Eine Auf- und Abwärtsbewegung entlang einer vertikal ausgerichteten Achse ist einfach zu realisieren und wenig störungsanfällig.

Insbesondere ist der Rotationsantrieb integraler Bestandteil der ersten Fördereinheit.

In einer weiteren vorteilhaften Ausgestaltung verlaufen die Richtung, in der das Laborgefäß in die Ringführung eingeführt wird, und die Verfahrrichtung in die Bereitstellungsposition entgegengesetzt zueinander. So muss das Laborgefäß zum Zentrieren und zum Verfahren in die Bereitstellungsposition nur entlang einer Achse bewegt werden. Dies vereinfacht die Konstruktion der Fördervorrichtung reduziert das Fehlerpotential und senkt die Kosten.

Vorzugsweise sind die Rotationsachse der Zentriervorrichtung und die vertikale Achse der ersten Fördereinheit identisch. Dies macht es einfacher, das Auflager, den Rotationsantrieb und die Achse der ersten Fördereinheit miteinander zu integrieren. Die Konstruktion der ersten Fördereinheit und der Zentriervorrichtung wird dadurch einfacher, weniger fehleranfällig und kostengünstiger.

Um insbesondere zu verhindern, dass falsch ausgerichtete Laborgefäße in den Analysebereich gefördert werden, ist mindestens ein Lagesensor dem Ausgangsbereich vorgeschaltet, der die Ausrichtung des Laborgefäßes im Hinblick auf oben und unten erfasst. Dadurch kann während des Transportprozesses Zeit eingespart werden, da Laborgefäße umgehend in den Ausgabebereich gefördert werden können, wenn ihre falsche Ausrichtung erfasst wurde. Wenn die Ausrichtung der zu transportierenden Laborgefäße für die Analyse/Präparation/Manipulation unerheblich ist, können die Lagesensoren einfach deaktiviert oder weggelassen werden.

Nach einem weiteren Aspekt der Erfindung ist das Laborgefäß rotationssymmetrisch ausgebildet und weist unterschiedliche Durchmesser über seine Höhe auf. Insbesondere weist das Laborgefäß einen nach oben offenen Behälter mit einem ersten Außendurchmesser und einen den Behälter verschließenden Deckel mit einem zweiten Außendurchmesser auf, wobei der zweite Außendurchmesser größer ist als der erste Außendurchmesser. Die verschiedenen großen Außendurchmesser erleichtern die Unterscheidung von Behälter und Deckel. Die rotationssymmetrische Ausbildung erleichtert die Handhabung des Laborgefäßes insgesamt, da bei der Eingabe, dem Ergreifen und der Ausgabe die Ausrichtung des Laborgefäßes bezüglich der horizontalen Ebene vernachlässigt werden kann. Dadurch wird die Fördervorrichtung effizienter und sicherer. Denkbar sind auch Fläschchen bzw. mit Flüssigkeit gefüllte, zylinderförmige Gefäße.

Günstig ist es, wenn ein Lagesensormodul in den Förderweg der dritten Fördereinheit eingebracht ist. Wenn vom Lagesensor ein falsch ausgerichtetes Laborgefäß erfasst wird, kann das Laborgefäß von der dritten Fördereinheit direkt zum Ausgabebereich gefördert werden, ohne zuvor die Zentriervorrichtung und/oder die erste Fördereinheit zu aktivieren. Dies spart Zeit und verbessert die Bedienung der Fördervorrichtung.

Es ist zweckmäßig, dass zwei quer zur Förderrichtung der dritten Fördereinheit ausgerichtete Lichtschranken vorgesehen sind, die jeweils einem Außendurchmesser des Laborgefäßes zugeordnet sind, so dass diejenige Lichtschranke zuerst ein Signal generiert, bei der der größere Außendurchmesser des Laborgefäßes zuerst eintritt, und dann zeitversetzt die andere Lichtschranke, bei der der kleinere Außendurchmesser eintritt, wodurch die Ausrichtung des Laborgefäßes bestimmbar ist. Diese Art der Bestimmung ist zuverlässig und kostengünstig, die Vorteile liegen unter anderem in einer berührungslosen Messung und der guten elektromagnetischen Verträglichkeit.

Da es in der gängigen Laborpraxis üblich ist, Laborgefäße, wie Petrischalen, zu deren eindeutiger Identifizierung mit Barcodes zu versehen, ist in einer erfindungsgemäßen Ausführungsform zumindest ein Barcode-Scanner vorgesehen. Um Barcodes verschiedener gängiger Formate sowohl auf der Seite als auch auf der Unterseite der Laborgefäße lesen zu können, ist es zweckmäßig, zwei Barcode-Scanner zu verwenden, die jeweils den Barcodes zugeordnet angeordnet sind. Über den Barcode werden die Laborgefäße eindeutig identifiziert und damit auch die Proben und deren Eigenschaften.

Vorzugsweise wird der Vorgang des Barcode-Lesens mit einer rotatorischen Bewegung um 360° der ersten Fördereinheit kombiniert. Diese Bewegung ermöglicht es, Barcodes unabhängig von der horizontalen Ausrichtung der Laborgefäße zu lesen.

Es ist zweckmäßig, nach erfolgreichem Lesen des Barcodes die rotatorische Bewegung zusätzlich um einen definierten Winkel weiterzuführen, um den seitlich angeordneten Barcode in eine definierte horizontale Lage zu bringen. Durch diese Ausrichtung kann eine eventuelle Beschädigung des Barcodes durch den Greifer der zweiten Fördereinheit verhindert werden.

Bei einer bevorzugten Ausführungsform ist die zweite Fördereinheit durch einen Transportarm mit einem Greifer am freien Ende ausgebildet. Transportarme sind vorteilhaft, um die Laborgefäße auch über größere Entfernungen zu fördern. Greifer sind gut geeignet zum Aufnehmen und Transportieren von Laborgefäßen, insbesondere Petrischalen, und können leicht an die unterschiedlichen Maße der Laborgefäße angepasst werden. Durch die Kombination aus Transportarm und Greifer werden die Einsatzmöglichkeiten der zweiten Fördereinheit und somit der Fördervorrichtung erweitert.

Vorzugsweise ist der Greifer im Transportarm drehbar gelagert und angetrieben, so dass dieser den ergriffenen Teil des Laborgefäßes in eine vorbestimmte Ausrichtung relativ zum Analysegerät, insbesondere um 180°, drehen und horizontal verfahren kann. Beispielsweise ist es üblich, Petrischalen so zu lagern, dass der Behälter oben angeordnet ist und der Deckel unten. Dabei weist die offene Seite des Behälters nach unten, unter anderem um während der Lagerung eine Ansammlung von Kondenswasser auf dem Nährmedium zu vermeiden. Analysegeräte wiederum sind häufig so aufgebaut, dass eine Erfassungseinheit, wie etwa eine Kamera, von oben auf die zu analysierenden Proben gerichtet ist. Daher ist eine Drehung des Laborgefäßes um 180° häufig erforderlich, um eine Analyse durchführen zu können. Die drehbare Ausführung des Greifers ermöglicht für einen derartigen Fall eine solche Drehung und vergrößert dadurch den Einsatzbereich der Fördervorrichtung.

Bei einer vorteilhaften Weiterbildung der Erfindung ist eine Absaugeinrichtung inklusive HEPA-Filter vorgesehen, welche zumindest zwischen dem Höhenbereich und dem Bereitstellungsbereich wirksam ist. Vor allem ist diese im Bereich der Separation von Deckel und Schale und der Rotation wirksam, da bei diesen Vorgängen gefährliche Partikel freiwerden könnten. So kann verhindert werden, dass gefährliche, krankheitserregende oder giftige Stoffe während der Analyse in die Umgebung gelangen. Der Einsatz der Fördervorrichtung wird dadurch deutlich sicherer.

Sehr günstig ist es weiterhin, wenn ein Sensor vorgesehen ist, der erfasst, wann das durch die erste Fördereinheit vertikal verfahrene Laborgefäß eine vorbestimmte Höhe erreicht hat. Diese Daten können zur Steuerung der ersten Fördereinheit verwendet werden, so dass das Laborgefäß abhängig von seiner Bauhöhe auf die vorbestimmte Höhe transportiert wird und damit exakt im erforderlichen Abstand von der Analyseeinheit gehalten wird. So können Laborgefäße verschiedener Maße von der zweiten Fördereinheit korrekt positioniert werden. Dies verbessert die Anwendungsmöglichkeiten der Fördervorrichtung.

Gemäß einem weiteren Aspekt wird die der Erfindung zugrunde liegende Aufgabe für das Verfahren dadurch gelöst, dass eine Fördervorrichtung zur Positionierung und Bereitstellung von Laborgefäßen für Proben, Mikroorganismen, Zellkulturen oder ähnlichem für die Analyse an einem zugeordneten Analysegerät, insbesondere wie sie vorstehend beschrieben wurde, verwendet wird, wobei vor dem Fördern durch die erste Fördereinheit in den Ausgangsbereich hinein die Ausrichtung des Laborgefäßes erfasst wird. Dies ist notwendig, wenn die Ausrichtung für die darauffolgenden Schritte relevant ist. Wenn die Ausrichtung der vorbestimmten Ausrichtung entspricht, wird das Laborgefäß im Ausgangsbereich zentriert. Wenn die Ausrichtung des Laborgefäßes jedoch nicht der vorbestimmten Ausrichtung entspricht, wird das Laborgefäß wieder aus dem Ausgangsbereich herausgefördert, insbesondere über die dritte Fördereinheit.

Dadurch wird sichergestellt, dass nur korrekt ausgerichtete und somit vom Gerät analysierbare, präparierbare oder manipulierbare Laborgefäße durch die erste Fördereinheit in das Analysegerät gefördert werden. Dadurch wird Zeit eingespart, und die Zahl nicht verwertbarer Ergebnisse wird reduziert. Die Effizienz der Analyse von Proben wird durch die vorgeschlagene Fördervorrichtung gesteigert.

Gemäß einem Aspekt der Erfindung wird das Laborgefäß vor dem Verfahren in den Höhenbereich relativ zur vertikalen Achse zentriert. Dadurch kann das Laborgefäß im Höhenbereich von der zweiten Fördereinheit bereits in einer vorbestimmten Position übernommen werden, von der aus durch die zweite Fördereinheit nur eine geringe Feinjustierung vorgenommen werden muss, um das Laborgefäß innerhalb des Analysegeräts exakt so zu positionieren, wie es für eine optimale Analyse erforderlich ist. Dadurch werden durch mangelhafte Positionierung des Laborgefäßes verursachte Ungenauigkeiten bei der Analyse vermieden und die zu erwartende Zuverlässigkeit des Analyseergebnisses weiter erhöht.

Vorzugsweise wird das Laborgefäß vor der Analyse geöffnet und nach der Analyse wieder verschlossen.

Es ist zweckmäßig, dass im Normalbetrieb über die dritte Fördereinheit die Laborgefäße aus dem Eingabebereich in den Ausgangsbereich gefördert werden, über die erste Fördereinheit die Laborgefäße aus dem Ausgangsbereich in den Höhenbereich und vice versa gefördert werden, über die zweite Fördereinheit die Laborgefäße in den Bereitstellungsbereich gefördert werden. Über dritte Fördereinheit wird das Laborgefäß von der Ausgangsposition weiter zum Beginn der Förderbänder der vierten Fördereinheit gefördert. Mittels der Förderbänder der vierten Fördereinheit wird das Laborgefäß dann an die gewünschte Position im Ausgabebereich transportiert. So wird zum einen eine maximale Präzision bei der Positionierung der Laborgefäße erreicht, da die einzelnen Fördereinheiten translatorische Bewegungen jeweils nur entlang einer Achse ausführen. Zum anderen können die Bewegungen der Fördereinheiten leicht synchronisiert werden, so dass in jedem Förderzyklus Zeit eingespart wird. Die Förderung der Laborgefäße wird dadurch sowohl sicherer als auch effizienter.

Nach einem bevorzugten Verfahren verbleiben über eine mit dem Lagesensor zusammenwirkende Steuereinheit nur vorbestimmt ausgerichtete Laborgefäße im Ausgangsbereich zum Verfahren in die Bereitstellungsposition, und alle anderen werden wieder aus dem Ausgangsbereich herausgefördert, insbesondere durch die dritte Fördereinheit. Dadurch wird sichergestellt, dass nur korrekt ausgerichtete und somit vom Analysegerät analysierbare Laborgefäße in das Analysegerät gefördert werden. Dadurch wird Zeit eingespart, und die Zahl nicht verwertbarer Ergebnisse wird reduziert. Die Effizienz der Förderung und Analyse von Proben wird durch die Verwendung der vorgeschlagenen Fördervorrichtung gesteigert.

Günstig ist es, wenn zumindest der analyserelevante oder zu präparierende Teil des Laborgefäßes aus dem Höhenbereich durch die zweite Fördereinheit ergriffen und dieser Teil des Laborgefäßes der Bereitstellungsposition zugeführt wird, während der Analyse gehalten und nach der Analyse wieder in die Bereitstellungsposition zurückgeführt wird. Dadurch entfällt die Notwendigkeit, in einem von der Förderung separaten Schritt den Deckel von dem Behälter abzunehmen und gegebenenfalls zwischenzulagern. Die Förderung wird dadurch schneller und effizienter, während gleichzeitig die Gefahr, einen Deckel zu verlieren, verringert wird.

Sehr vorteilhaft ist es außerdem, wenn in der Zentriervorrichtung das Laborgefäß unter Rotation des Auflagers mit dem Laborgefäß eingeführt wird. Durch die Rotationsbewegung wird das Laborgefäß langsam und gleichmäßig zentriert, wodurch verhindert wird, dass das Laborgefäß in Schieflage gerät und schlimmstenfalls verkantet. Dadurch wird die Sicherheit der Zentrierung und der Förderung verbessert.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: eine Perspektivansicht einer erfindungsgemäßen Fördervorrichtung;
- Fig. 2: eine seitliche Schnittansicht eines zentralen Bereichs der Fördervorrichtung, der einen Ausgangsbereich und einen Höhenbereich umfasst; und
- Fig. 3: eine Flussdiagramm, in dem ein möglicher Ablauf eines Verfahrens nach der beanspruchten Erfindung dargestellt ist.

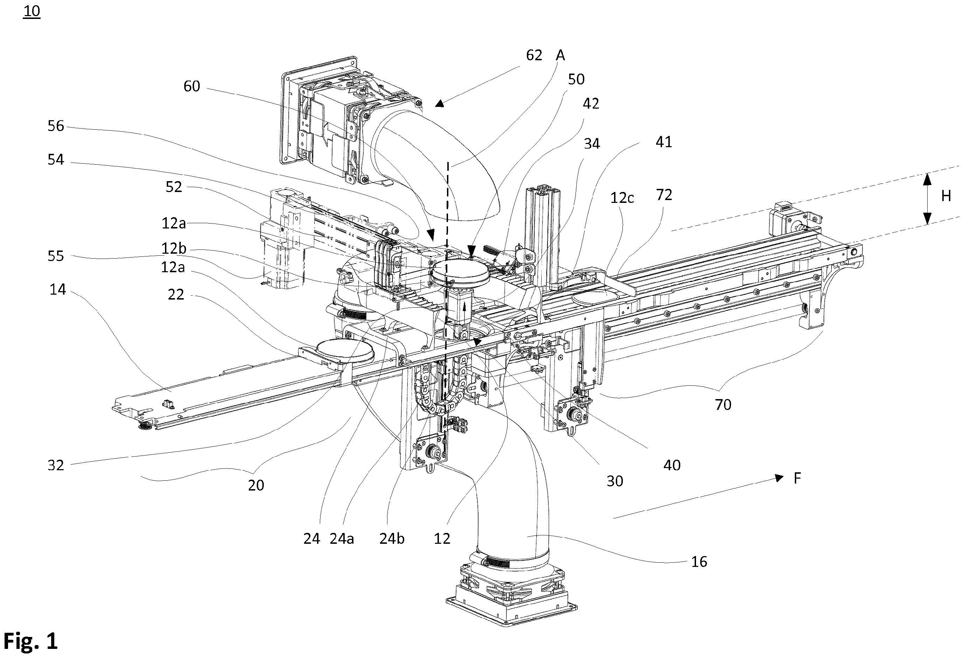

Fig. 1 zeigt eine Perspektivansicht einer erfindungsgemäßen Fördervorrichtung 10 ohne Befestigungen, Verbindungen mit einem Gehäuse oder Schnittstellen zu anderen Modulen/Komponenten eines möglichen Gesamtsystems. In der Fördervorrichtung 10 werden auf einer Förderbahn 14 Laborgefäße, insbesondere Petrischalen 12, gefördert.

Unterhalb der Förderbahn 14 ist eine s-förmige Ansaugeinheit 16 mit einem Filter vorgesehen, um mögliche, beim Öffnen der Petrischalen austretende Keime, Bakterien u. ä. sofort abzutransportieren und eine Kontamination der Umgebung und der Fördervorrichtung 10 auf alle Fälle zu verhindern. Die Ansaugeinheit 16 saugt Umgebungsluft an, diese wird gefiltert und in Richtung der Petrischale 12 ausgestoßen. Gegenüber dem oberen Ende der Ansaugeinheit 16 ist eine Ansaugeinrichtung 62 vorgesehen, welche die aus der Ansaugeinheit 16 ausgestoßene Luft, welche um die Petrischale 12 herumströmt und dadurch einen Strömungswand bildet, welche ein Austreten von Keimen verhindert, ansaugt. In der Ansaugeinrichtung 62 ist ein HEPA-Filter eingebracht, welche die Luft keimfrei filtert. Die gefilterte Luft wird dann wieder aus der Ansaugeinrichtung 62 an die Umgebung ausgestoßen. Der Luftstrom verläuft somit von unten nach oben.

Die Petrischalen 12 sind in herkömmlicher Weise zweiteilig ausgeführt und umfassen einen Behälter 12a und einen den Behälter 12a bereichsweise umgreifenden und verschließenden Deckel 12b. Die Förderbahn 14 weist vom Betrachter aus gesehen links einen Eingabebereich 20 auf, in dem die Petrischalen 12 manuell oder automatisiert in die Fördervorrichtung 10 eingebracht werden. Ein Pusher 22 verschiebt die Petrischale 12 in einen vom Betrachter aus gesehen rechts an den Eingabebereich 20 angrenzenden Ausgangsbereich 30 der Förderbahn 14, der später im Detail noch erläutert wird.

An den Ausgangsbereich 30 wiederum schließt ein Ausgabebereich 70 einer weiteren Förderbahn 15 an, in dem die Petrischalen 12 manuell oder automatisiert aus der Fördervorrichtung 10 entnommen werden. Im Ausgabebereich 70 ist eine Schranke 72 vorgesehen, die verhindert, dass die Petrischalen 12 zu weit transportiert werden. Die Petrischalen 12 werden von der Schranke 72 gestoppt. Über ein Förderband der weiteren Förderbahn 15 werden die Petrischalen 12 vom Ausgangsbereich 30 weg zu einer vom Ausgangsbereich 30 entfernt liegenden Stelle des Ausgabebereichs 70 transportiert.

Aus dieser Anordnung ergibt sich eine globale Förderrichtung F, in die sich der Pusher 22 und das Förderband der Förderbahn 15 mit einer Petrischale 12 bewegen.

Der Pusher 22 übergreift die Förderbahn 14 in der Art einer Klammer an einer Seite und wird in herkömmlicher Weise von einem unter der Förderbahn 14 angeordneten Elektromotor in oder gegen die Förderrichtung F angetrieben. Der Pusher 22 ist in der vorliegenden Anordnung ausschließlich der Förderbahn 14 zugeordnet.

Die weitere Förderbahn 15 umfasst ein aus zwei Riemen bestehendes Förderband. Das Förderband ist nicht durchgehend ausgeführt, damit eine sich daran anschließende, hier nicht dargestellte, Sortiereinheit die Petrischale 12 auch vertikal nach oben fördern kann.

In der Fig. 1 ist der Pusher 22 zusammen mit einer Petrischale 12 im Eingabebereich 20 dargestellt. Auf der Förderbahn 14 ist zwischen Eingabebereich 20 und Ausgangsbereich 30 ein Lagesensormodul 24 angeordnet, der mit einer zentralen, hier nicht dargestellten Steuereinheit verbunden ist. Dieses Lagesensormodul 24 umfasst zwei vertikal übereinander angeordnete Lichtschranken 24a und 24b, die so voneinander beabstandet sind, dass die obere Lichtschranke 24a den oben lagernden Teil 12a oder 12b einer das Lagesensormodul 24 passierenden Petrischale 12 erfasst und die untere Lichtschranke 24b den unten lagernden Teil 12b oder 12a der Petrischale 12.

Auf Grund des unterschiedlichen Durchmessers von Behälter 12a und Deckel 12b der Petrischale 12 ist durch den Lagesensor 24 die Ausrichtung der Petrischale 12 erfassbar. Der Deckel 12b der Petrischale 12 umgreift den Behälter 12a bereichsweise und weist daher einen größeren Durchmesser auf als der Behälter 12a. Folglich erfasst die erste der beiden Lichtschranken 24a und 24b, die zuerst unterbrochen wird, das Passieren des Deckels 12b und die zweite das Passieren des Behälters 12a.

Für die hier beschriebene Ausführungsform der vorliegenden Erfindung ist es erforderlich, dass die Petrischalen 12 mit dem Deckel 12b nach unten und dem Behälter 12a nach oben eingebracht werden. Zum einen werden Petrischalen 12 häufig in dieser Ausrichtung gelagert. Zum anderen ist diese Ausrichtung von Petrischalen 12 beim Verarbeiten von Proben vorteilhaft, da der Behälter 12a leicht abgenommen und nach dem Verarbeiten wieder aufgesetzt werden kann und ein zusätzlicher Schritt entfällt, bei dem der Deckel 12b abgenommen, während des Verarbeitens zwischengelagert und anschließend wieder aufgesetzt wird. Hierauf wird später bei der Erläuterung des beanspruchten Verfahrens näher eingegangen.

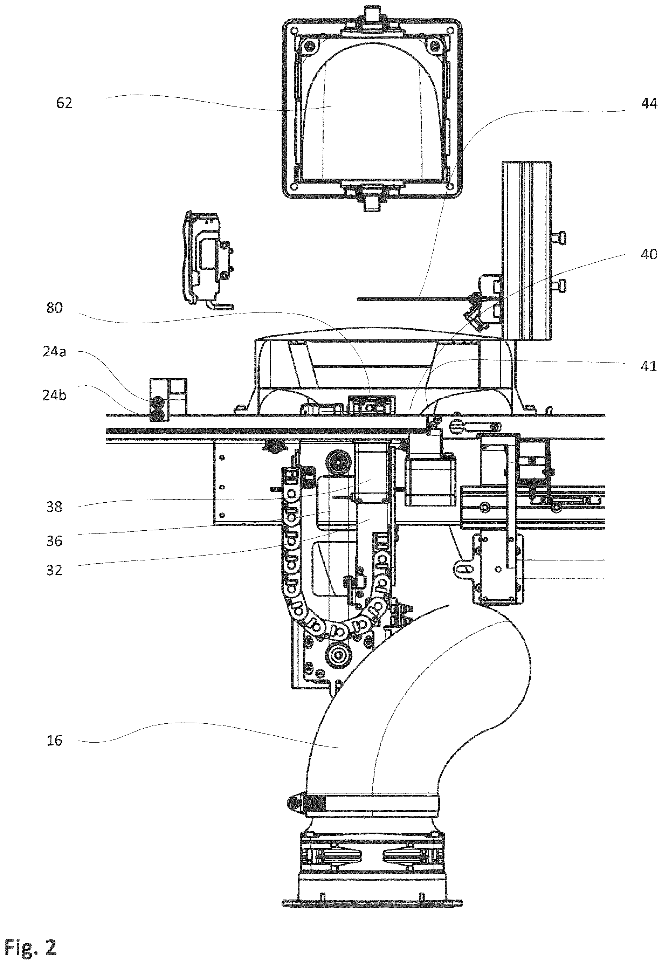

Im Ausgangsbereich 30 ist in die Förderbahn 14 eine kreisförmige Ausnehmung 40 eingebracht, die eine sich konisch nach unten verjüngende seitliche Ringführung 41 aufweist, siehe Figur 2. Die Ausnehmung 40 wird von einer entlang einer Achse A vertikal bewegbaren Liftvorrichtung 32 durchgriffen. Die Liftvorrichtung 32 umfasst einen Antrieb 36 für die vertikale Verfahrbewegung und einen um die Achse A rotierbaren Auflageteller 34. Der Durchmesser des Auflagetellers 34 ist so bemessen, dass er die Ausnehmung 40 der Förderbahn 14 an der Stelle ihres geringsten Durchmessers mit minimalem Spiel passieren kann. Unterhalb des Auflagetellers 34 ist ein elektrischer Antrieb 38 für eine Rotationsbewegung des Auflagetellers 34 angeordnet.

Der mit der Liftvorrichtung 32 vertikal verschiebbare und rotierbare Auflageteller 34 und die Ausnehmung 40 mit ihrer sich konisch verjüngenden seitlichen Ringführung 41 bilden zusammen eine Zentriervorrichtung für eine exakte Zentrierung der Petrischalen 12. Der Zentriervorgang wird später im Zusammenhang mit dem beanspruchten Verfahren beschrieben.

In der Fig. 1 ist eine exakt auf dem Auflagenteller 34 zentrierte Petrischale 12 dargestellt, wobei sich der Auflagenteller 34 mitsamt der Petrischale 12 oberhalb der Förderbahn 14 in einem Höhenbereich 50 befindet.

In einer Ebene mit dem Höhenbereich 50 befindet sich ein Bereitstellungsbereich für die nachfolgende Analyse/Probenmanipulation/Probenpräparation durch ein hier nicht dargestelltes Gerät, beispielsweise ein Analysegerät für die in dem Petrischalenbehälter 12a befindlichen Proben.

Die Petrischale 12, genauer gesagt der oben auf dem Deckel 12b aufliegende Behälter 12a, wird von einer Greifvorrichtung 52 in dem Höhenbereich 50 ergriffen, anschließend wird der Auflageteller 34 zumindest um den Radius und die Höhe des Deckels zuzüglich eines Sicherheitsabstandes nach unten verfahren, insbesondere ganz nach unten. Der Greifer 56 trägt nun nur den Behälter 12a. Die Greifer 56 dreht den Behälter 12b um 180° und verfährt diesen translatorisch in einen Bereitstellungsbereich. Im Bereitstellungsbereich wird die in dem Behälter 12a befindliche Probe dann von einem hier nicht dargestellten Analysegerät analysiert. Die Petrischalen 12 bzw. Behälter 12a der Petrischale 12 sind im Bereitstellungsbereich in der separaten Analyse- oder Präparationseinheit angeordnet.

Die Greifvorrichtung 52 weist einen Trägerarm 54 und den am freien Ende des Trägerarms 54 angeordneten Greifer 56 auf. Dabei ist ein Antrieb 55 vorgesehen, durch den der der Trägerarm 54 orthogonal zur Förderrichtung F vorwärts und zurück bewegbar ist. Der Greifer 56 ist mit einem Antrieb 58 versehen, mit dem er um die Längsachse des Trägerarms 54 gedreht werden kann. Durch die translatorische Bewegung des Trägerarms 54 sind Petrischalenbehälter 12a translatorisch zwischen dem Höhenbereich 50 und einer Analyseeinheit förderbar. Ferner ist die Ausrichtung einer ergriffenen Petrischale 12 bzw. eines Teils einer Petrischale 12 im Hinblick auf oben und unten durch eine rotatorische Bewegung des Greifers 56 änderbar. Dies wird nachfolgend im Zusammenhang mit dem Verfahren näher erläutert.

Im Ausgabebereich 70 ist in der Fig. 1 eine weitere Petrischale 12c dargestellt. Der Förderrichtung F entsprechend hat die Petrischale 12c den Analysevorgang bereits durchlaufen ist bereit für die Entnahme aus dem Ausgabebereich 70.

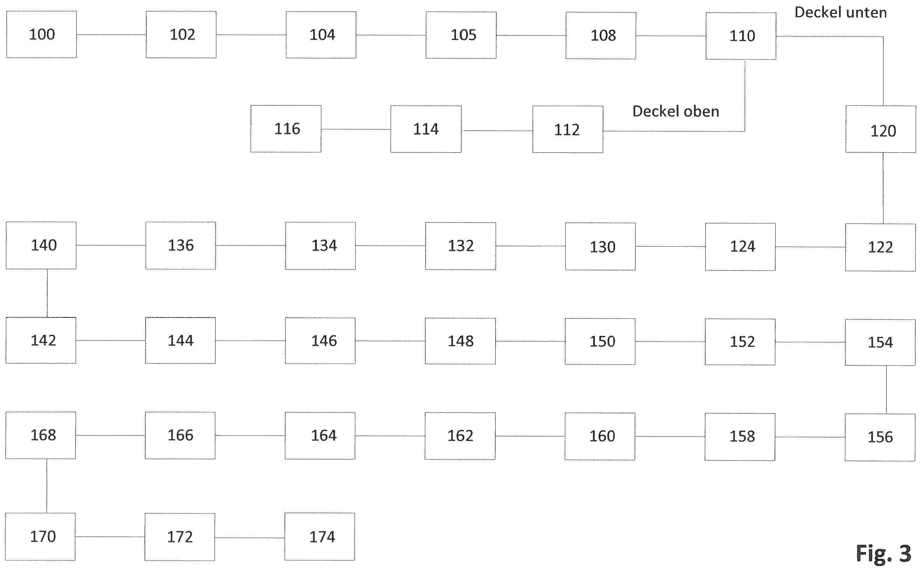

Fig. 3 zeigt ein Flussdiagramm, in dem ein möglicher Ablauf eines Verfahrens nach der beanspruchten Erfindung dargestellt ist. Hierdurch wird auch das Zusammenwirken der zuvor beschriebenen Elemente der Fördervorrichtung 10 nochmals verdeutlicht.

Nach dem Einbringen 100 einer Petrischale 12 in den Eingabebereich 20 der Fördervorrichtung 10 fördert 102 der Pusher 22 die Petrischale 12 entlang der Förderbahn 14 in Richtung des Ausgangsbereichs 30. Die Petrischale 12 passiert 104 zwischen dem Eingabebereich 20 und dem Ausgangsbereichs 30 den Lagesensor 24, der mit der Steuereinheit verbunden ist. Da der Außendurchmesser des Deckels 12b der Petrischale 12 größer ist als der des Behälters 13a, generiert 106 diejenige der beiden übereinander angeordneten Lichtschranken 24a und 24b des Lagesensors 24 zuerst ein Signal, die der Ebene des Deckels 12b zugeordnet ist.

Das zuerst generierte 106 Signal wird von der Steuereinheit ausgewertet 110. Zugleich wird die Liftvorrichtung 32 durch den Antrieb 36 vertikal soweit verfahren 108, dass der Auflageteller 34 und die Förderbahn 14 in einer Ebene liegen. Nach dem erfindungsgemäßen Verfahren ist es erforderlich, dass die Petrischale 12 mit dem Deckel 12b unten und dem Behälter 12a oben eingebracht wird. Ergibt die Auswertung 110 durch die Steuereinheit, dass das zuerst generierte 106 Signal von der Lichtschranke 24a stammt und folglich der Deckel 13b nach oben ausgerichtet ist, wird die Verarbeitung der Petrischale 12 abgebrochen 112. Der Pusher 22 verfährt 114 mitsamt der Petrischale 12 über den Ausgangsbereich 30 hinweg bis das Förderband der weiteren Förderbahn 15, dann übernimmt das Förderband den Transport zum Ausgabebereich 70. Sodann verfährt 116 der Pusher 22 zurück in den Eingabebereich und ist bereit für die Förderung 102 einer weiteren Petrischale 12.

Ergibt die Auswertung 108 durch die Steuereinheit, dass das zuerst generierte 106 Signal von der Lichtschranke 24b stammt und folglich der Deckel 12b nach unten ausgerichtet ist, verfährt 120 der Pusher 22 mitsamt der Petrischale 12 in den Ausgangsbereich 30, bis die Petrischale 12 auf dem Auflageteller 34 lagert 122. Der Pusher 22 verfährt 124 zurück in den Eingabebereich 20 und ist bereit für die Förderung 102 einer weiteren Petrischale 12.

Zur exakten Zentrierung der Petrischale 12 wird der Auflageteller 34 mitsamt der Petrischale 12 vom Antrieb 38 in Rotation versetzt 130. Zugleich beginnt die Liftvorrichtung 32, nach unten zu verfahren 132. Bei der Abwärtsbewegung entlang der sich nach unten konisch verjüngenden Ringführung 41 der in den Ausgangsbereich 30 eingebrachten Ausnehmung 40 wird die auf dem Auflageteller 34 lagernde Petrischale 12 zentriert und lagert 134 schließlich vollständig auf der Ringführung 41.

Die Liftvorrichtung 32 verfährt 136 weiter nach unten, bis der Auflageteller 34 sich vollständig unterhalb der Ringführung 41 befindet. Dadurch wird gewährleistet, dass sich die Petrischale 12 zentriert auf der Ringführung 41 lagert.

Der Barcode-Scanner 44 liest den Barcode am Laborgefäß 12 aus 140 und die Auswerteeinheit 46 wertet den Barcode aus 142. Dieser Vorgang 140 des Barcode-Lesens erfolgt vor dem Anheben der Petrischalen 12. Der Barcode ist entweder an der Unterseite der Petrischale 12 aufgeklebt, wie in diesem Fall, wenn man darauf blickt oben, da die Schalen invertiert sind, oder an der Seite der Petrischale 12. Um Barcodes an der Unterseite und Seite gleichermaßen erfassen zu können, sind zwei Barcodes nötig. Im Falle eines seitlichen Barcodes wird die Petrischale 12 von der Rotationsvorrichtung 38 um maximal 180° rotiert, bis der seitlich angeordnete Barcode-Scanner 80 den Barcode erfasst hat. Ab Erfassen des Barcodes wird die Rotationsbewegung noch um einen definierten Winkel fortgeführt, um den seitlichen Barcode nach vorne auszurichten und damit zu verhindern, dass die Spitzen des Greifers 56 den Barcode beschädigen könnten.

Anschließend fährt die Liftvorrichtung 32 wieder nach oben 144und nimmt die Petrischale 12 auf 146. Mitsamt der auf dem Auflageteller 34 lagernden Petrischale 12 verfährt die Liftvorrichtung 32 weiter 148, bis der Höhensensor 42 die Petrischale 12 in einer vorbestimmten Höhe im Höhenbereich 50 erfasst 150, ein Signal an die zentrale Steuereinheit sendet 152 und die zentrale Steuereinheit daraufhin die Liftvorrichtung 32 zum Anhalten der Verfahrbewegung ansteuert 154.

Daraufhin steuert die zentrale Steuereinheit die Greifvorrichtung 52 an 156 zum Ergreifen des Petrischalenbehälters 12a und anschließendem Verfahren der Liftvorrichtung 32 um einen vorbestimmten Wert nach unten 158. Anschließend wird der Antrieb 55 der Greifvorrichtung 52 aktiviert 160, um den Behälter 12a aus dem Höhenbereich 50 in den Bereitstellungsbereich der Analyse- oder Präparationseinheit.

Während des Verfahrens des Trägerarms 54 mit dem Behälter 12a in den Bereitstellungsbereich der Analyse- oder Präparationsbereich dreht 162 der Antrieb den Greifer 56 samt Behälter 12a um 180°, so dass die offene Seite des Behälters 12a nach oben gewandt ist. Der Greifer 56 ist ohne äußere Einwirkung durch Federkraft geschlossen, um den Petrischalenbehälter 12a während des Analyse- oder Präparationsvorganges zuverlässig halten zu können, ohne dass ein motorischer Antrieb notwendig ist. Geöffnet wird dieser über einen weiteren Motor.

Nach Abschluss der Analyse 164 verfährt der Trägerarm 54 mit dem Behälter 13a wieder von der Analyse- oder Präparationseinheit 64 in den Höhenbereich 50, und zugleich dreht 166 der Antrieb den Greifer 56 samt Behälter 12a erneut um 180° in die ursprüngliche Ausrichtung.

Ist der Greifer 56 in den Höhenbereich 50 verfahren, verfährt 168 die Liftvorrichtung 32 mit dem Deckel 12b unter den Behälter 12a, und der Greifer 56 gibt der Behälter 12a auf den auf dem Auflageteller 34 lagernden Deckel 12b frei 170. Die Liftvorrichtung 32 fährt nun mit dem Laborgefäß 12 nach unten 172 bis zum bündigen Abschluss des Auflagetellers 34 mit Förderbahn 14.

Zuletzt fördern 174 die Förderbänder der Förderbahn 15 das Laborgefäß 12 in den Ausgabebereich 70, wo es manuell oder automatisiert entnommen werden kann 172.

### Bezugszeichenliste

- 10: Fördervorrichtung
- 12: Petrischale
- 12a: Behälter
- 12b: Deckel
- 14: Förderbahn
- 15: weitere Förderbahn
- 16: Ansaugeinheit
- 20: Eingabebereich
- 22: Pusher
- 24: Lagesensor
- 24a, b: Lichtschranken
- 30: Ausgangsbereich
- 32: Liftvorrichtung
- 34: Auflageteller
- 36: vertikaler Antrieb
- 38: Rotationsantrieb
- 40: Ausnehmung
- 41: Ringführung
- 42: Höhensensor
- 44: Barcode-Scanner
- 46: Auswerteeinheit
- 50: Höhenbereich
- 52: Greifvorrichtung
- 54: Trägerarm
- 55: Antrieb (translatorisch)
- 56: Greifer
- 60: Bereitstellungsbereich
- 62: Absaugeinrichtung
- 70: Ausgabebereich
- 72: Schranke
- 80: seitlicher Barcodescanner
- 100: Einbringen eines Laborgefäßes 12 in den Eingabebereich 20
- 102: Pusher 22 fördert das Laborgefäß 12 in Richtung Ausgangsbereich 30
- 104: Laborgefäß 12 passiert Lagesensor 24
- 106: Lagesensor 24 sendet Signal an zentrale Steuereinheit
- 108: Liftvorrichtung 32 wird vom Antrieb 36 bis zum bündigen Abschluss mit Förderbahn 14 verfahren
- 110: Steuereinheit wertet Signal des Lagesensors 24 aus
- 112: Abbruch der Verarbeitung des Laborgefäßes 12
- 114: Pusher 22 verfährt mit Laborgefäß zum Ausgabebereich 70
- 116: Pusher 22 verfährt zurück in Eingabebereich 20
- 120: Pusher 22 fördert das Laborgefäß 12 in Ausgangsbereich 30 hinein
- 122: Laborgefäß 12 lagert auf Auflageteller 34
- 124: Pusher 22 verfährt zurück in Eingabebereich 20
- 130: Antrieb 38 versetzt Auflageteller 34 in Rotation
- 132: Liftvorrichtung 32 verfährt nach unten
- 134: Laborgefäß 12 lagert auf Ringführung 41
- 136: Liftvorrichtung 32 verfährt bis unterhalb der Ausnehmung 40
- 140: Barcode-Scanner 44 liest Barcode am Laborgefäß 12 aus
- 142: Auswerteeinheit 46 wertet Barcode aus
- 144: Liftvorrichtung 32 verfährt nach oben
- 146: Auflageteller 34 nimmt Laborgefäß 12 auf
- 148: Liftvorrichtung 32 verfährt mit Laborgefäß 12 nach oben
- 150: Höhensensor 42 erfasst Laborgefäß 12
- 152: Höhensensor 42 sendet Signal an zentrale Steuereinheit
- 154: zentrale Steuereinheit steuert Liftvorrichtung 32 zum Anhalten an
- 156: Ergreifen des Petrischalenbehälters 12a
- 158: Verfahren der Liftvorrichtung 32 um einen vorbestimmten Wert nach unten
- 160: Verfahren des Behälters 12a aus dem Höhenbereich 50 in den Bereitstellungsbereich
- 162: Antrieb dreht Greifer 56 samt Behälter 12a um 180°
- 164: Trägerarm 54 verfährt mit Behälter 12a aus der Analyseeinheit 64
- 166: Antrieb dreht Greifer 56 samt Behälter 12a um 180°
- 168: Verfahren der Liftvorrichtung unter den Behälter 12a
- 170: Greifer 56 gibt Behälter 12a auf Auflageteller 34 frei
- 172: Liftvorrichtung 32 fährt mit Pertrischale 12 nach unten bis zum bündigen Abschluss des Auflagetellers 34 mit Förderbahn 14
- 174: Förderband der weiteren Förderbahn 15 fördert Laborgefäß 12 in Ausgabebereich 70

- A: Achse
- H: vorbestimmte Höhe
- F: Förderrichtung

## Patentansprüche

1. Fördervorrichtung (10) zur Positionierung und Bereitstellung von Laborgefäßen (12; 12a, 12b, 12c) für Nährböden, Proben, Mikroorganismen, Zellkulturen oder ähnlichem für die Analyse, Probenpräparation und/oder Probenmanipulation an einem zugeordneten Gerät (64), mit zumindest einer ersten Fördereinheit (32) zum Zu- und/oder Abfördern der Laborgefäße (12) aus einem Ausgangsbereich (30) zu einem Bereitstellungsbereich (60), wobei die erste Fördereinheit (32) das Laborgefäß (12) aus dem Ausgangsbereich (30) zu einem vorbestimmten Höhenbereich (50) und vice versa vertikal fördert, und eine zweite Fördereinheit (52) vorgesehen ist, welche das Laborgefäß (12) aus dem Höhenbereich (50) in den Bereitstellungsbereich (60) und vice versa horizontal fördert, wobei die Fördereinheiten jeweils lediglich eine translatorische Bewegung des Laborgefäßes (12) entlang einer Achse (A) durchführen, **dadurch gekennzeichnet, dass** die zweite Fördereinheit aus dem Höhenbereich (50) das Laborgefäß in den Bereitstellungsbereich (60) und vice versa horizontal fördert, im Bereitstellungsbereich (60) das Laborgefäß (12) für die Analyse oder Präparation gehalten wird, eine Zentriervorrichtung (34, 40) vorgesehen ist, welche das Laborgefäß (12) relativ zur Achse (A) der ersten Fördereinheit (32) mittig zentriert, die erste Fördereinheit (32) ein Auflager (34) für das Laborgefäß (12) aufweist, das horizontal ausgerichtet ist, und dass sich die Achse (A) der ersten Fördereinheit (32) orthogonal zur Auflagerfläche erstreckt und die Zentriervorrichtung (34, 40) einen Rotationsantrieb (38) für das Auflager (34) und eine seitliche Ringführung (41) für das Laborgefäß (12) umfasst, welche sich konisch in eine Richtung verkleinert, so dass zum Zentrieren des Laborgefäßes dieses auf dem Auflager (34) um die Achse (A) der ersten Fördereinheit gedreht wird, das Auflager (34) in Richtung der konischen Verkleinerung verfahren wird, dabei von der seitlichen Ringführung (41) das Laborgefäß (12) geführt wird und dadurch das Laborgefäß (12) zur Achse (A) auf dem Auflager (34) zentriert wird.

2. Fördervorrichtung nach Anspruch 1, **dadurch gekennzeichnet, dass** eine dritte Fördereinheit (14, 22) vorgesehen ist, welche ein Laborgefäß (12) aus einem Eingabebereich (20) zu dem Ausgangsbereich (30) horizontal fördert und/oder dass eine vierte Fördereinheit (14, 72) vorgesehen ist, welche ein Laborgefäß (12) aus dem Ausgangsbereich (30) zu einem Ausgabebereich (70) horizontal fördert.

3. Fördervorrichtung nach einem der vorangehenden Ansprüche, **dadurch gekennzeichnet, dass** das Auflager (34) nach unten und oben entlang der vertikal ausgerichteten Achse (A) verfahrbar ist und/oder dass der Rotationsantrieb (38) integraler Bestandteil der ersten Fördereinheit (32) ist.

4. Fördervorrichtung nach einem der vorangehenden Ansprüche, **dadurch gekennzeichnet, dass** die Richtung, in der das Laborgefäß (12) in die Ringführung (41) eingeführt wird, und die Verfahrrichtung in den Höhenbereich (50) entgegengesetzt zueinander verlaufen.

5. Fördervorrichtung nach Anspruch 3 oder 4, **dadurch gekennzeichnet, dass** die Rotationsachse der Zentriervorrichtung (34, 40) und die vertikale Achse (A) der ersten Fördereinheit (32) identisch sind.

6. Fördervorrichtung nach einem der vorangehenden Ansprüche, **dadurch gekennzeichnet, dass** ein Lagesensor (24) dem Ausgangsbereich (30) vorgeschaltet ist, der die Ausrichtung des Laborgefäßes (12) im Hinblick auf oben und unten erfasst, vorzugsweise dass das Laborgefäß (12) rotationssymmetrisch ausgebildet ist und unterschiedliche Durchmesser über seine Höhe aufweist, das Laborgefäß (12), insbesondere einen nach oben offenen Behälter (12a) mit einem ersten Außendurchmesser und einen den Behälter (12a) verschließenden Deckel (12b) mit einem zweiten Außendurchmesser aufweist, wobei der zweite Außendurchmesser des Deckels (12b) größer ist als der erste Außendurchmesser des Behälters (12a).

7. Fördervorrichtung nach Anspruch 5 und 6, wobei eine dritte Fördereinheit (14, 22) gemäß Anspruch 2 vorgesehen ist, **dadurch gekennzeichnet, dass** der Lagesensor (24) in den Förderweg der dritten Fördereinheit (14, 22) eingebracht ist, insbesondere dass zwei quer zur Förderrichtung F der dritten Fördereinheit (14, 22) ausgerichtete Lichtschranken (24a, 24b) vorgesehen sind, die jeweils einem Außendurchmesser des Laborgefäßes (12) zugeordnet sind, so dass diejenige Lichtschranke (24a, 24b) zuerst ein Signal generiert, bei der der größere Außendurchmesser des Laborgefäßes (12b) zuerst eintritt, und dann zeitversetzt die andere Lichtschranke, bei der der kleinere Außendurchmesser des Laborgefäßes (12a) eintritt, wodurch die Ausrichtung des Laborgefäßes (12) bestimmbar ist.

8. Fördervorrichtung nach einem der vorangehenden Ansprüche, **dadurch gekennzeichnet, dass** die zweite Fördereinheit (52) durch einen Transportarm (54) mit einem Greifer (56) am freien Ende ausgebildet ist, insbesondere dass der Greifer (56) im Transportarm (54) drehbar gelagert und angetrieben (55) ist, so dass dieser den ergriffenen Teil (12a) des Laborgefäßes (12) in eine vorbestimmte Ausrichtung relativ zum Analysegerät (64), insbesondere um 180°, drehen und horizontal verfahren kann.

9. Fördervorrichtung nach einem der vorangehenden Ansprüche, **dadurch gekennzeichnet, dass** eine Absaugeinrichtung (62) vorgesehen ist, welche zumindest zwischen dem Höhenbereich (50) und dem Bereitstellungsbereich (60) wirksam ist.

10. Fördervorrichtung nach einem der vorangehenden Ansprüche, **dadurch gekennzeichnet, dass** ein Sensor (42) vorgesehen ist, der erfasst, wann das durch die erste Fördereinheit (32) vertikal verfahrene Laborgefäß (12) eine vorbestimmte Höhe (H) erreicht hat.

11. Verfahren zur Positionierung und Bereitstellung von Laborgefäßen (12) für Proben mit einer Fördervorrichtung (10) nach einem der vorangehenden Ansprüche, **dadurch gekennzeichnet, dass** vor dem Fördern durch die erste Fördereinheit (32) in den Ausgangsbereich (30) hinein die Ausrichtung des Laborgefäßes (12) erfasst wird, wobei wenn die Ausrichtung der vorbestimmten Ausrichtung entspricht, das Laborgefäß (12) im Ausgangsbereich (30) zentriert wird und, wenn die Ausrichtung des Laborgefäßes (12) nicht der vorbestimmten Ausrichtung entspricht, das Laborgefäß (12) wieder aus dem Ausgangsbereich (30) herausgefördert wird, insbesondere über die vierte Fördereinheit (14, 72) gemäß Anspruch 2, vorzugsweise dass das Laborgefäß (12) vor dem Verfahren in den Höhenbereich (50) relativ zur vertikalen Achse (A) zentriert wird.

12. Verfahren nach Anspruch 11, **dadurch gekennzeichnet, dass** das Laborgefäß (12) vor der Analyse geöffnet und nach der Analyse wieder verschlossen wird.

13. Verfahren nach Anspruch 2 und nach einem der Ansprüche 11 oder 12, **dadurch gekennzeichnet, dass** im Normalbetrieb über die dritte Fördereinheit (14, 22) die Laborgefäße (12) in den Ausgangsbereich (30) gefördert werden, über die erste Fördereinheit (32) die Laborgefäße (12) aus dem Ausgangsbereich (30) in den Höhenbereich (50) gefördert werden, über die zweite Fördereinheit (52) die Laborgefäße (12) in den Bereitstellungsbereich (60) gefördert werden und über die vierte Fördereinheit (15) die Laborgefäße (12) aus dem Ausgangsbereich (30) in den Ausgabebereich (70) gefördert werden.

14. Verfahren nach einem der Ansprüche 11 bis 13, **dadurch gekennzeichnet, dass** über eine mit dem Lagesensor (24) zusammenwirkende Steuereinheit (80) nur vorbestimmt ausgerichtete Laborgefäße (12) im Ausgangsbereich (30) zum Verfahren in den Bereitstellungsbereich verbleiben, und alle anderen Laborgefäße (12) wieder aus dem Ausgangsbereich (30) herausgefördert werden, insbesondere durch die dritte Fördereinheit (14, 22) gemäß Anspruch 2.

15. Verfahren nach einem der Ansprüche 11 bis 14, **dadurch gekennzeichnet, dass** zumindest ein Teil (12a) des Laborgefäßes (12) aus dem Höhenbereich (50) durch die zweite Fördereinheit (52) ergriffen und dieser Teil (12a) des Laborgefäßes (12) dem Bereitstellungsbereich (60) zugeführt wird, dieser Teil (12a) während der Analyse gehalten und nach der Analyse wieder in die Bereitstellungsbereich (60) zurückgeführt wird.

16. Verfahren nach einem der Ansprüche 11 bis 15, **dadurch gekennzeichnet, dass** in der Zentriervorrichtung (34, 40) das Laborgefäß (12) unter Rotation des Auflagers (34) mit dem Laborgefäß (12) eingeführt wird.

## Claims

1. Conveying device (10) for the positioning and the provision of laboratory vessels (12; 12a, 12b, 12c) for nutrient media, samples, microorganisms, cell cultures, or the like for analysis, sample preparation, and/or sample manipulation at an associated apparatus (64), having at least one first conveying unit (32) for conveying said laboratory vessels (12) backward and forward between an initial region (30) and a provision region (60), which first conveying unit (32) vertically conveys said laboratory vessel (12) from said initial region (30) to a predetermined height region (50) and vice versa; and in which a second conveying unit (52) is provided that horizontally conveys said laboratory vessel (12) from said height region (50) to said provision region (60) and vice versa, said conveying units each only performing a translational movement of said laboratory vessel (12) along an axis (A), **characterized in that** said second conveying unit horizontally conveys said laboratory vessel from said height region (50) to said provision region (60) and vice versa, said laboratory vessel (12) is retained in said provision region (60) for the intended analysis or preparation, a centering device (34, 40) is provided that acts to center said laboratory vessel (12) relative to said axis (A) of said first conveying unit (32), said first conveying unit (32) comprises a horizontally aligned support (34) for said laboratory vessel (12), and **in that** said axis (A) of said first conveying unit (32) extends orthogonally with respect to the support surface, and that said centering device (34, 40) comprises a rotational drive (38) for said support (34) and a lateral ring guide (41) for said laboratory vessel (12), which ring guide (41) tapers conically in one direction, so that for centering said laboratory vessel the latter is rotated on the support (34) about said axis (A) of said first conveying unit, said support (34) is moved in the direction of the conical taper, in which process said laboratory vessel (12) is guided by said lateral ring guide (41), thus centering the laboratory vessel (12) on the support (34) relative to said axis (A).

2. Conveying device according to claim 1, **characterized in that** a third conveying unit (14, 22) is provided which horizontally conveys a laboratory vessel (12) from an input region (20) to said initial region (30), and/or **in that** a fourth conveying unit (14, 72) is provided which horizontally conveys a laboratory vessel (12) from said initial region (30) to an output region (70).

3. Conveying device according to any one of the preceding claims, **characterized in that** said support (34) can be moved up and down along said vertically aligned axis (A) and/or that said rotational drive (38) is an integral component of said first conveying unit (32).

4. Conveying device according to any one of the preceding claims, **characterized in that** the direction in which said laboratory vessel (12) is introduced into said ring guide (41) and the direction of travel in said height region (50) are opposite to each other.

5. Conveying device according to one of claims 3 and 4 above, **characterized in that** said axis of rotation of said centering device (34, 40) and said vertical axis (A) of said first conveying unit (32) are identical.

6. Conveying device according to any one of the preceding claims, **characterized in that** a position sensor (24) is connected upstream of said initial region (30) and is adapted to detect the orientation of said laboratory vessel (12) with respect to top and bottom, preferably **in that** said laboratory vessel (12) is of a rotationally symmetrical design and has different diameters along its height, said laboratory vessel (12) has a container (12a), which is in particular open to the top and has a first outer diameter, and a lid (12b) closing said container (12a), which has a second outer diameter, with said second outer diameter of said lid (12b) being larger than said first outer diameter of said container (12a).

7. Conveying device according to one of claims 5 and 6 above, wherein a third conveyor unit (14, 22) is provided according to claim 2, **characterized in that** said position sensor (24) is mounted in the conveying path of said third conveying unit (14, 22), in particular **in that** two light barriers (24a, 24b) are provided which are aligned in a direction transverse to the conveying direction, F, of said third conveying unit (14, 22) and that are each assigned to an outer diameter of said laboratory vessel (12), so that the light barrier (24a, 24b) at which the larger outer diameter of said laboratory vessel (12b) enters first will be the first to generate a signal, and then, with some time delay, the other light barrier at which the smaller outer diameter of said laboratory vessel (12a) enters, will generate a signal, thus allowing the determination of the orientation of said laboratory vessel (12).

8. Conveying device according to any one of the preceding claims, **characterized in that** said second conveying unit (52) is formed by a transport arm (54) having a gripper (56) at its free end, which gripper (56) is in particular rotatably mounted and driven (55) in said transport arm (54), thus allowing it to rotate, in particular by 180°, and horizontally move the gripped part (12a) of said laboratory vessel (12) into a predetermined orientation relative to an analysis device (64).

9. Conveying device according to any one of the preceding claims, **characterized in that** a suction device (62) is provided, which is effective at least between said height region (50) and said provision region (60).

10. Conveying device according to any one of the preceding claims, **characterized in that** a sensor (42) is provided which detects when said laboratory vessel (12) that has been vertically moved by said first conveying unit (32) has reached a predetermined height (H).

11. Method for positioning and providing laboratory vessels (12) for samples by means of a conveying device (10) according to any one of the preceding claims, **characterized in that** that before said laboratory vessel (12) is conveyed by said first conveying unit (32) into the initial region (30), the orientation of said laboratory vessel (12) is first detected, whereupon, if said orientation corresponds to the predetermined orientation, said laboratory vessel (12) is centered in said initial region (30), and if the orientation of said laboratory vessel (12) does not correspond to the predetermined orientation, said laboratory vessel (12) is conveyed out of said initial region (30) again, in particular by means of said fourth conveying unit (14, 72) according to claim 2, preferably **in that** said laboratory vessel (12) is centered relative to said vertical axis (A) before being moved into the height region (50).

12. Method according to claim 11, **characterized in that** said laboratory vessel (12) is opened before the analysis and closed again after the analysis.

13. Method according to claim 2 and according to one of claims 11 and 12 above, **characterized in that** in normal operation, said third conveying unit (14, 22) is used to convey said laboratory vessels (12) into said initial region (30), said first conveying unit (32) is used to convey said laboratory vessels (12) from said initial region (30) to said height region (50), said second conveying unit (52) is used to convey said laboratory vessels (12) into said provision region (60), and said fourth conveying unit (15) is used to convey said laboratory vessels (12) from said initial region (30) into said output area (70).

14. Method according to one of claims 11 to 13 above, **characterized in that** a control unit (80) which cooperates with said position sensor (24) is used to ensure that only laboratory vessels (12) of a predetermined orientation remain in the initial region (30) so as to be moved into the provision region, and all other laboratory vessels (12) are conveyed out of said initial region (30) again, in particular by means of said third conveying unit (14, 22) according to claim 2.

15. Method according to one of claims 11 to 14 above, **characterized in that** at least a part (12a) of said laboratory vessel (12) is grasped from said height region (50) by said second conveying unit (52) and this part (12a) of said laboratory vessel (12) is then conveyed to said provision region (60), which part (12a) is retained during the analysis and returned again to said provision region (60) after the analysis.

16. Method according to one of claims 11 to 15 above, **characterized in that** said laboratory vessel (12) is introduced into said centering device (34, 40) by rotating said support (34) with said laboratory vessel (12).

## Revendications

1. Dispositif de transport (10) pour le positionnement et la fourniture de récipients de laboratoire (12 ; 12a, 12b, 12c) pour des milieux de culture, échantillons, micro-organismes, cultures cellulaires ou similaires pour l'analyse, la préparation d'échantillons et/ou manipulation d'échantillons sur un appareil (64) associé, avec au moins une première unité de transport (32) pour l'amenée et/ou l'évacuation des récipients de laboratoire (12) à partir d'une zone de départ (30) vers une zone de fourniture (60), dans lequel la première unité de transport (32) transporte verticalement le récipient de laboratoire (12) à partir de la zone de départ (30) vers une zone en hauteur (50) prédéfinie et vice versa, et une deuxième unité de transport (52) est prévue, laquelle transporte horizontalement le récipient de laboratoire (12) à partir de la zone en hauteur (50) dans la zone de fourniture (60) et vice versa, dans lequel les unités de transport effectuent respectivement uniquement un mouvement en translation du récipient de laboratoire (12) le long d'un axe (A), **caractérisé en ce que** la deuxième unité de transport transporte horizontalement le récipient de laboratoire à partir de la zone en hauteur (50) dans la zone de fourniture (60) et vice versa, le récipient de laboratoire (12) est retenu dans la zone de fourniture (60) pour l'analyse ou la préparation, un dispositif de centrage (34, 40) est prévu, lequel centre de manière centrale le récipient de laboratoire (12) par rapport à l'axe (A) de la première unité de transport (32), la première unité de transport (32) présente un support (34) pour le récipient de laboratoire (12), qui est orienté horizontalement, et que l'axe (A) de la première unité de transport (32) s'étend perpendiculairement à la surface de support et le dispositif de centrage (34, 40) comprend un entraînement en rotation (38) pour le support (34) et un guidage annulaire latéral (41) pour le récipient de laboratoire (12), lequel diminue de manière conique dans une direction, de sorte que pour le centrage du récipient de laboratoire, celui-ci est amené en rotation sur le support (34) autour de l'axe (A) de la première unité de transport, le support (34) est déplacé en direction de la diminution conique, ce faisant le récipient de laboratoire (12) est guidé par le guidage annulaire latéral (41) et de cette manière le récipient de laboratoire (12) est centré par rapport à l'axe (A) sur le support (34).

2. Dispositif de transport selon la revendication 1, **caractérisé en ce qu'**une troisième unité de transport (14, 22) est prévue, laquelle transporte horizontalement un récipient de laboratoire (12) à partir d'une zone d'entrée (20) vers la zone de départ (30) et/ou qu'une quatrième unité de transport (14, 72) est prévue, laquelle transporte horizontalement un récipient de laboratoire (12) à partir de la zone de départ (30) vers une zone de sortie (70).

3. Dispositif de transport selon l'une quelconque des revendications précédentes, **caractérisé en ce que** le support (34) est déplaçable vers le bas et le haut le long de l'axe (A) orienté verticalement et/ou que l'entraînement en rotation (38) fait partie intégrante de la première unité de transport (32).

4. Dispositif de transport selon l'une quelconque des revendications précédentes, **caractérisé en ce que** la direction dans laquelle le récipient de laboratoire (12) est introduit dans le guidage annulaire (41) et la direction de déplacement dans la zone en hauteur (50) s'étendent de manière opposée l'une à l'autre.

5. Dispositif de transport selon la revendication 3 ou 4, **caractérisé en ce que** l'axe de rotation du dispositif de centrage (34, 40) et l'axe vertical (A) de la première unité de transport (32) sont identiques.

6. Dispositif de transport selon l'une quelconque des revendications précédentes, **caractérisé en ce qu'**un capteur de position (24) est monté en amont de la zone de départ (30), qui détecte l'orientation du récipient de laboratoire (12) en ce qui concerne le haut et le bas, de préférence que le récipient de laboratoire (12) est réalisé à symétrie de rotation et présente différents diamètres sur sa hauteur, le récipient de laboratoire (12) présente en particulier un contenant (12a) ouvert vers le haut avec un premier diamètre extérieur et un couvercle (12b) fermant le contenant (12a) avec un deuxième diamètre extérieur, dans lequel le deuxième diamètre extérieur du couvercle (12b) est plus grand que le premier diamètre extérieur du contenant (12a).

7. Dispositif de transport selon la revendication 5 et 6, dans lequel une troisième unité de transport (14, 22) est prévue selon la revendication 2, **caractérisé en ce que** le capteur de position (24) est inséré dans la voie de transport de la troisième unité de transport (14, 22), en particulier que deux barrières lumineuses (24a, 24b) orientées transversalement par rapport à la direction de transport F de la troisième unité de transport (14, 22) sont prévues, qui sont associées respectivement à un diamètre extérieur du récipient de laboratoire (12), de sorte que la barrière lumineuse (24a, 24b) au niveau de laquelle le plus grand diamètre extérieur du récipient de laboratoire (12b) entre en premier, génère un signal en premier, et ensuite de manière décalée dans le temps l'autre barrière lumineuse, au niveau de laquelle le plus petit diamètre extérieur du récipient de laboratoire (12a) entre, moyennant quoi l'orientation du récipient de laboratoire (12) peut être déterminée.

8. Dispositif de transport selon l'une quelconque des revendications précédentes, **caractérisé en ce que** la deuxième unité de transport (52) est réalisée par un bras de transport (54) avec un élément de préhension (56) sur l'extrémité libre, en particulier que l'élément de préhension (56) est monté en rotation dans le bras de transport (54) et entraîné (55) de sorte que celui-ci peut amener en rotation et déplacer horizontalement la partie (12a) saisie du récipient de laboratoire (12) dans une orientation prédéfinie par rapport à l'appareil d'analyse (64), en particulier de 180°.

9. Dispositif de transport selon l'une quelconque des revendications précédentes, **caractérisé en ce qu'**un système d'aspiration (62) est prévu, lequel est actif au moins entre la zone en hauteur (50) et la zone de fourniture (60).

10. Dispositif de transport selon l'une quelconque des revendications précédentes, **caractérisé en ce qu'**un capteur (42) est prévu, qui détecte quand le récipient de laboratoire (12) déplacé verticalement par la première unité de transport (32) a atteint une hauteur (H) prédéfinie.

11. Procédé pour le positionnement et la fourniture de récipients de laboratoire (12) pour des échantillons avec un dispositif de transport (10) selon l'une quelconque des revendications précédentes, **caractérisé en ce qu'**avant le transport par la première unité de transport (32) à l'intérieur de la zone de départ (30) l'orientation du récipient de laboratoire (12) est détectée, dans lequel lorsque l'orientation correspond à l'orientation prédéfinie, le récipient de laboratoire (12) est centré dans la zone de départ (30) et, lorsque l'orientation du récipient de laboratoire (12) ne correspond pas à l'orientation prédéfinie, le récipient de laboratoire (12) est transporté à nouveau à l'extérieur de la zone de départ (30), en particulier par l'intermédiaire de la quatrième unité de transport (14, 72) selon la revendication 2, de préférence que le récipient de laboratoire (12) est centré par rapport à l'axe vertical (A) avant le déplacement dans la zone en hauteur (50).

12. Procédé selon la revendication 11, **caractérisé en ce que** le récipient de laboratoire (12) est ouvert avant l'analyse et refermé après l'analyse.

13. Procédé selon la revendication 2 et selon l'une quelconque des revendications 11 ou 12, **caractérisé en ce qu'**en fonctionnement normal les récipients de laboratoire (12) sont transportés dans la zone de départ (30) par l'intermédiaire de la troisième unité de transport (14, 22), les récipients de laboratoire (12) sont transportés par l'intermédiaire de la première unité de transport (32) hors de la zone de départ (30) dans la zone en hauteur (50), les récipients de laboratoire (12) sont transportés dans la zone de fourniture (60) par l'intermédiaire de la deuxième unité de transport (52) et les récipients de laboratoire (12) sont transportés hors de la zone de départ (30) dans la zone de sortie (70) par l'intermédiaire de la quatrième unité de transport (15).

14. Procédé selon l'une quelconque des revendications 11 à 13, **caractérisé en ce que** par l'intermédiaire d'une unité de commande (80) coopérant avec le capteur de position (24), seuls les récipients de laboratoire (12) orientés de manière prédéfinie restent dans la zone de départ (30) pour le déplacement dans la zone de fourniture, et tous les autres récipients de laboratoire (12) sont à nouveau sortis de la zone de départ (30), en particulier par la troisième unité de transport (14, 22) selon la revendication 2.

15. Procédé selon l'une quelconque des revendications 11 à 14, **caractérisé en ce qu'**au moins une partie (12a) du récipient de laboratoire (12) est saisie à partir de la zone en hauteur (50) par la deuxième unité de transport (52) et cette partie (12a) du récipient de laboratoire (12) est amenée à la zone de fourniture (60), cette partie (12a) est maintenue pendant l'analyse et après l'analyse ramenée dans la zone de fourniture (60).

16. Procédé selon l'une quelconque des revendications 11 à 15, **caractérisé en ce que** le récipient de laboratoire (12) est introduit dans le dispositif de centrage (34, 40) sous l'effet d'une rotation du support (34) avec le récipient de laboratoire (12).
